# EUROPEAN PATENT APPLICATION

(11) **EP 4 840 485 A1**
(43) Date of publication of application: **18.11.2026**
(21) Application number: 26178406.0
(22) Date of filing: 13.05.2026
(51) Int. Cl.: A61M 1/36, A61M 1/02, A61M 1/26, A61M 1/34, A61M 1/38, G01N 15/01, G01N 15/06

(54) **AUTOMATIC ADJUSTMENT OF LIGHT INTENSITY FOR OPTICAL DETECTION OF BLOOD CELL CONCENTRATION**

(30) Priority: 15.05.2025 US 202563806050 P
(71) Applicant: Fenwal, Inc., Lake Zurich, IL 60047 (US)
(72) Inventor: KUSTERS, Benjamin E., Lake Zurich, 60047 (US); MIN, Kyungyoon, Lake Zurich, 60047 (US); MADSEN, James G., Lake Zurich, 60047 (US)
(74) Representative: Maikowski & Ninnemann Patentanwälte Partnerschaft mbB

(57) **Abstract**

A blood processing device includes a pump system, a valve system, a controller, and an optical detection assembly having a light source and a light detector and being configured to be controlled by the controller to monitor a fluid during a blood processing procedure. The controller is programmed to determine an expected range of cellular concentrations for the fluid during the procedure and to determine or be provided with an intensity of light to be emitted by the light source during the procedure based on the expected range of cellular concentrations. The controller controls the light source during the procedure to emit light at that intensity toward the fluid and controls the light detector to transmit a signal to the controller based on light from the light source received by the light detector, with the controller determining a cellular concentration of the fluid based on the signal.

## Description

### Background

### Field of the Disclosure

The present disclosure relates to determination of the concentration of blood cells in a fluid. More particularly, the present disclosure relates to optical detection of the concentration of blood cells in a fluid and automatic adjustment of the intensity of light emitted by the light source of an optical detection assembly.

### Description of Related Art

Various blood processing systems now make it possible to collect particular blood constituents, instead of whole blood, from a blood source. Typically, in such systems, whole blood is drawn from a blood source, the particular blood component or constituent is separated, removed, and collected, and the remaining blood constituents are returned to the blood source. Removing only particular constituents is advantageous when the blood source is a living subject (e.g., a human or animal), because potentially less time is needed for their body to return to pre-procedure levels, thus allowing for more iterations of a procedure (e.g., a platelet donation procedure) to be repeated in a given amount of time for the source compared to when whole blood is collected. This increases the overall supply of blood constituents, such as plasma and platelets, made available for transfer and/or therapeutic treatment.

It is known to employ an optical detection assembly to determine the concentration of blood cells within a fluid during a blood processing procedure. This may include, for example, detecting the hematocrit or concentration of red blood cells in packed red blood cells separated from whole blood, detecting the concentration of platelets in platelet-rich plasma separated from whole blood, or detecting the concentration of platelets in a platelet concentrate separated from platelet-rich plasma. An exemplary optical detection assembly is described in U.S. Patent Application Publication No. 2023/0243746 A1, which is hereby incorporated herein by reference.

It has been found that the concentration of cells within a fluid being monitored by an optical detection assembly may impact the intensity of light to be used by the optical detection assembly for optimal determination of the concentration of cells within the fluid. For example, light having a relatively low intensity may be appropriate for monitoring a fluid having a relatively low blood cell concentration, whereas the same light intensity may be inappropriate for monitoring a fluid having a greater blood cell concentration. Accordingly, it would be advantageous to provide an optical detection assembly that is configured for optimal determination of the concentration of blood cells in fluids having different blood cell concentrations.

### Summary

There are several aspects of the present subject matter which may be embodied separately or together in the devices and methods described and claimed below. These aspects may be employed alone or in combination with other aspects of the subject matter described herein, and the description of these aspects together is not intended to preclude the use of these aspects separately or the claiming of such aspects separately as set forth in the claims appended hereto.

In one aspect, a blood processing device includes a pump system, a valve system, a controller programmed to control the operation of the pump system and the valve system to execute a blood processing procedure, and an optical detection assembly. The optical detection assembly includes a light source and a light detector and is configured to be controlled by the controller to monitor a fluid during the blood processing procedure. The controller is programmed to determine an expected range of cellular concentrations for the fluid and to determine or be provided with an intensity of light to be emitted by the light source during the blood processing procedure based at least in part on the expected range of cellular concentrations for the fluid. The controller controls the light source during the blood processing procedure to emit light at that intensity toward the fluid and controls the light detector to transmit a signal to the controller based on light from the light source received by the light detector, with the controller determining a cellular concentration of the fluid based at least in part on the signal from the light detector.

In another aspect, a controller-implemented method is provided for executing a blood processing procedure using a blood processing device comprising a pump system, a valve system, and an optical detection assembly comprising a light source and a light detector and configured to be controlled by the controller to monitor a fluid during the blood processing procedure. The method includes the controller determining an expected range of cellular concentrations for the fluid and then determining or being provided with an intensity of light to be emitted by the light source during the blood processing procedure based at least in part on the expected range of cellular concentrations for the fluid. The controller controls the light source during the blood processing procedure to emit light at that intensity toward the fluid and controls the light detector to transmit a signal to the controller based on light from the light source received by the light detector, with the controller determining a cellular concentration of the fluid based at least in part on the signal from the light detector.

### Brief Description of the Drawings

Fig. 1 is a perspective view of an exemplary hardware component or blood processing device of a blood processing system according to an aspect of the present disclosure;
Fig. 2 is a schematic view of an exemplary disposable component or fluid flow circuit mounted to the hardware component of Fig. 1 to complete a blood processing system according to an aspect of the present disclosure;
Fig. 3 illustrates the blood processing system of Fig. 2 implementing a draw phase of an exemplary blood processing procedure;
Fig. 4 is a schematic view of an optical detection assembly of the blood processing device of Fig. 1, monitoring a fluid having a relatively low concentration of cellular blood components;
Fig. 5 illustrates the optical detection assembly of Fig. 4 monitoring a fluid having a relatively high concentration of cellular blood components using light with a relatively low intensity;
Fig. 6 illustrates the optical detection assembly of Fig. 4 monitoring a fluid having a relatively high concentration of cellular blood components using light with a relatively high intensity;
Fig. 7 is a flowchart illustrating an exemplary approach to automatic adjustment of the intensity of light emitted by the light source of an optical detection assembly during a blood processing procedure, according to an aspect of the present disclosure;
Fig. 8 is a schematic view of an alternative embodiment of an optical detection assembly of the blood processing device of Fig. 1; and
Fig. 9 is a schematic view of another alternative embodiment of an optical detection assembly of the blood processing device of Fig. 1.

### Description of the Illustrated Embodiments

The embodiments disclosed herein are for the purpose of providing an exemplary description of the present subject matter. They are, however, only exemplary, and the present subject matter may be embodied in various forms. Therefore, specific details disclosed herein are not to be interpreted as limiting the subject matter as defined in the accompanying claims.

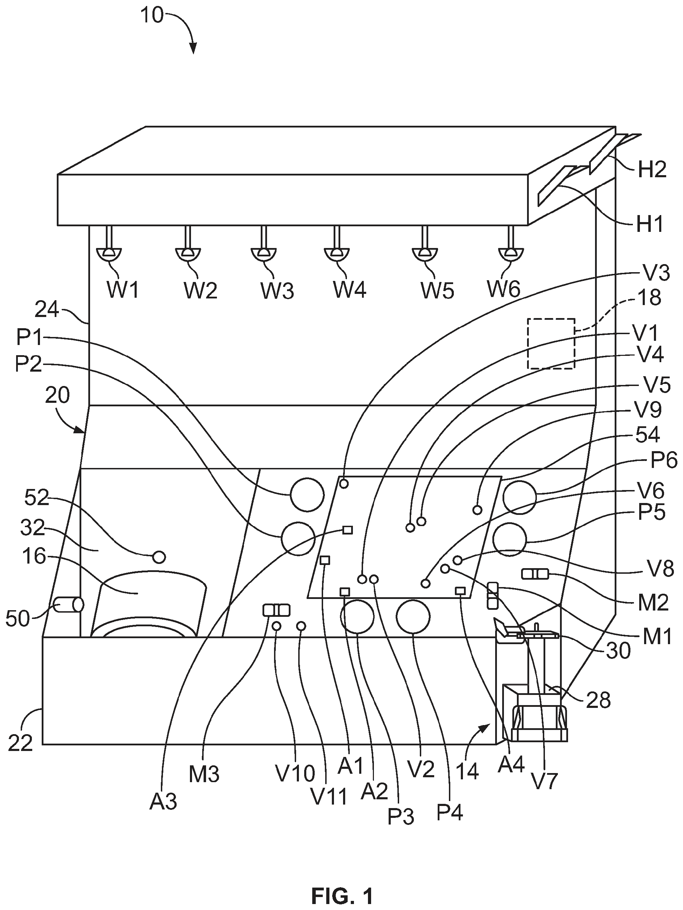

Figs. 1-6 show components of a blood processing system that embodies various aspects of the present subject matter. Use of the system for separating blood into two or more components and collecting at least one of the components (namely, platelets) will be described herein, though it should be understood that systems according to the present disclosure can be used for processing a variety of different biological fluids.

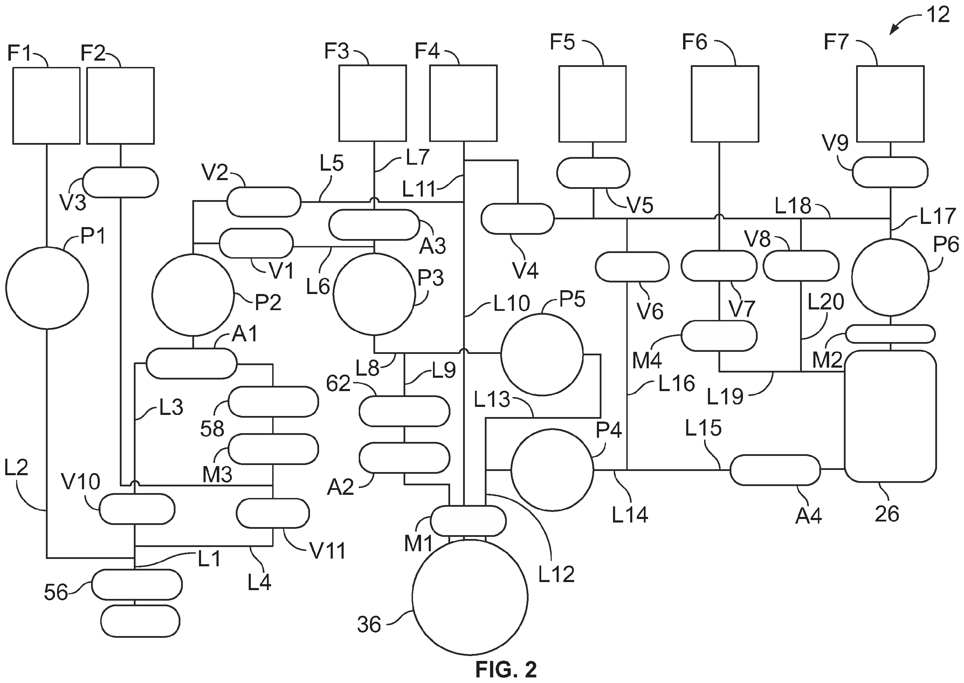

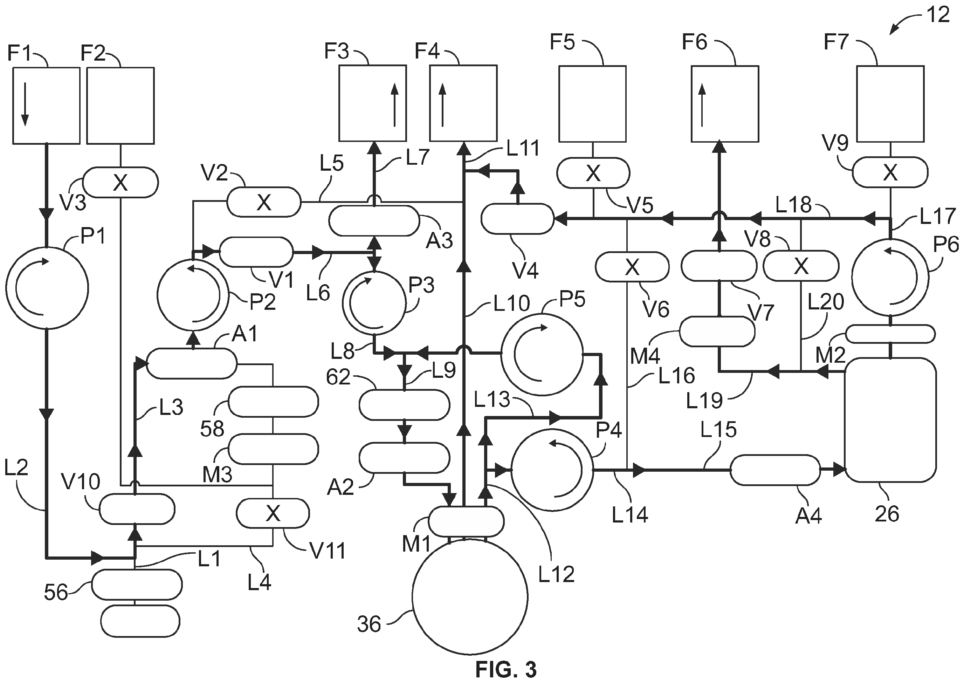

Generally speaking, the system includes two principal components, a durable and reusable blood processing device 10 (Fig. 1) and a disposable fluid flow circuit 12 (Figs. 2 and 3). The illustrated blood processing system is configured generally in accordance with the system described in PCT Patent Application Publication No. WO 2018/053217 A1, which is hereby incorporated herein by reference. The blood processing device 10 of Fig. 1 includes a spinning membrane separator drive unit 14, a centrifuge or centrifugal separator 16, additional components that control fluid flow through the disposable fluid flow circuit 12, and a controller 18, which governs the operation of the other components of the blood processing device 10 to perform a blood processing procedure. While the principles described herein may be employed when using the blood processing device 10 of Fig. 1, it should be understood that these same principles may be applied to other blood processing devices, including devices employing single separation technologies or approaches.

### I. The Durable Blood Processing Device

The blood processing device 10 (Fig. 1) is configured as a durable item that is capable of long-term use. It should be understood that the blood processing device 10 of Fig. 1 is merely exemplary of one possible configuration and that blood processing devices according to the present disclosure may be differently configured.

In the illustrated embodiment, the blood processing device 10 is embodied in a single housing or case 20. The illustrated case 20 includes a generally horizontal portion 22 (which may include an inclined or angled face or upper surface for enhanced visibility and ergonomics) and a generally vertical portion 24. The spinning membrane separator drive unit 14 and the centrifugal separator 16 are shown as being incorporated into the generally horizontal portion 22 of the case 20, while the controller 18 is shown as being incorporated into the generally vertical portion 24.

### A. Spinning Membrane Separator Drive Unit

The blood processing device 10 includes a spinner support or spinning membrane separator drive unit 14 for accommodating a generally cylindrical spinning membrane separator 26 of the fluid flow circuit 12 (Figs. 2 and 3). U.S. Patent No. 5,194,145 (which is hereby incorporated herein by reference) describes an exemplary spinning membrane separator drive unit that would be suitable for incorporation into the blood processing device 10, but it should be understood that the spinning membrane separator drive unit 14 may be differently configured without departing from the scope of the present disclosure.

The illustrated spinning membrane separator drive unit 14 has a base 28 configured to receive a lower portion of the spinning membrane separator 26 and an upper end cap 30 to receive an upper portion of the spinning membrane separator 26. Preferably, the upper end cap 30 is positioned directly above the base 28 to orient a spinning membrane separator 26 received by the spinning membrane separator drive unit 14 vertically and to define a vertical axis about which the spinning membrane separator 26 is spun. While it may be advantageous for the spinning membrane separator drive unit 14 to vertically orient a spinning membrane separator 26, it is also within the scope of the present disclosure for the spinning membrane separator 26 to be differently oriented when mounted to the blood processing device 10.

In one embodiment, one of the base 28 and upper end cap 30 of the spinning membrane separator drive unit 14 is movable with respect to the other, which may allow differently sized spinning membrane separators 26 to be received by the spinning membrane separator drive unit 14. For example, the upper end cap 30 may be translated vertically with respect to the base 28 and locked in a plurality of different positions, with each locking position corresponding to a differently sized spinning membrane separator 26.

At least one of the base 28 and the upper end cap 30 is configured to spin one or more components of the spinning membrane separator 26 about the axis defined by the spinning membrane separator drive unit 14. The mechanism by which the spinning membrane separator drive unit 14 spins one or more components of the spinning membrane separator 26 may vary without departing from the scope of the present disclosure. In one embodiment, a component of the spinning membrane separator 26 to be spun includes at least one element configured to be acted upon by a magnet (e.g., a metallic material), while the spinning membrane separator drive unit 14 includes a magnet (e.g., a series of magnetic coils or semicircular arcs). By modulating the magnetic field acting upon the aforementioned element of the spinning membrane separator 26, the component or components of the spinning membrane separator 26 may be made to spin in different directions and at varying speeds. In other embodiments, different mechanisms may be employed to spin the component or components of the spinning membrane separator 26.

Regardless of the mechanism by which the spinning membrane separator drive unit 14 spins the component or components of the spinning membrane separator 26, the component or components of the spinning membrane separator 26 is/are preferably spun at a speed that is sufficient to create Taylor vortices in a gap between the spinning component and a stationary component of the spinning membrane separator 26 (or a component that spins at a different speed). Fluid to be separated within the spinning membrane separator 26 flows through this gap, and filtration may be dramatically improved by the creation of Taylor vortices.

### B. Centrifugal Separator

As for the centrifugal separator 16, it includes a centrifuge compartment 32 that receives a centrifugal separation chamber 36 of the fluid flow circuit 12 (Figs. 2 and 3), as well as other components of the centrifugal separator 16. Further details as to an exemplary configuration of the centrifugal separator are set forth in PCT Patent Application Publication No. WO 2018/053217 A1.

Fluid (e.g., anticoagulated whole blood) is introduced into the centrifugal separation chamber 36 by an umbilicus, with the fluid being separated into a layer of less dense components (e.g., platelet-rich plasma, in the case of blood being separated) and a layer of more dense components (e.g., packed red blood cells) within the centrifugal separation chamber 36 as a result of centrifugal forces as it rotates. Components of an interface monitoring system may be positioned within the centrifuge compartment 32 to oversee separation of fluid within the centrifugal separation chamber 36. The interface monitoring system may include a light source 50 and a light detector 52, which is positioned and oriented to receive at least a portion of the light emitted by the light source 50.

The orientation of the various components of the interface monitoring system depends at least in part on the particular configuration of the centrifugal separation chamber 36. In general, though, the light source 50 emits a light beam (e.g., a laser light beam) through the separated fluid components within the centrifugal separation chamber 36 (which may be formed of a material that substantially transmits the light or at least a particular wavelength of the light without absorbing it). A portion of the light reaches the light detector 52, which transmits a signal to the controller 18 that is indicative of the location of an interface between the separated fluid components. If the controller 18 determines that the interface is in the wrong location (which can affect the separation efficiency of the centrifugal separator 16 and/or the quality of the separated fluid components), then it can issue commands to the appropriate components of the blood processing device 10 to modify their operation so as to move the interface to the proper location.

### C. Other Components Of The Blood Processing Device

In addition to the spinning membrane separator drive unit 14 and the centrifugal separator 16, the blood processing device 10 may include other components compactly arranged to aid fluid processing.

The generally horizontal portion 22 of the case 20 of the illustrated blood processing device 10 includes a cassette station 54, which accommodates a flow control cassette of the fluid flow circuit 12. In one embodiment, the cassette station 54 is similarly configured to the cassette station of U.S. Patent No. 5,868,696 (which is hereby incorporated herein by reference), but is adapted to include additional components and functionality. The illustrated cassette station 54 includes a plurality of clamps or valves V1-V9 (which are collectively referred to herein as the "valve system" of the blood processing system 10), which move between a plurality of positions (e.g., between a retracted or lowered position and an actuated or raised position) to selectively contact or otherwise interact with corresponding valve stations of the flow control cassette of the fluid flow circuit 12. Depending on the configuration of the fluid flow circuit 12, its cassette may not include a valve station for each valve V1-V9 of the cassette station 54, in which case fewer than all of the valves V1-V9 will be used in a fluid processing procedure.

In the actuated position, a valve V1-V9 engages the associated valve station to prevent fluid flow through that valve station (e.g., by closing one or more ports associated with the valve station, thereby preventing fluid flow through that port or ports). In the retracted position, a valve V1-V9 is disengaged from the associated valve station (or less forcefully contacts the associated valve station than when in the actuated position) to allow fluid flow through that valve station (e.g., by opening one or more ports associated with the valve station, thereby allowing fluid flow through that port or ports). Additional clamps or valves V10 and V11 of the valve system may be positioned outside of the cassette station 54 to interact with portions of valve stations (which may be lengths of tubing) of the fluid flow circuit 12 to selectively allow and prevent fluid flow therethrough. The valves V1-V9 and corresponding valve stations of the cassette station 54 and cassette may be differently configured and operate differently from the valves V10 and V11 and the valve stations that are spaced away from the cassette station 54.

The cassette station 54 may be provided with additional components, such as pressure sensors A1-A4, which interact with sensor stations of the cassette to monitor the pressure at various locations of the fluid flow circuit 12. For example, if the fluid source is a human donor, one or more of the pressure sensors A1-A4 may be configured to monitor the pressure of the donor's vein during blood draw and return. Other pressure sensors A1-A4 may monitor the pressure of the spinning membrane separator 26 and the centrifugal separation chamber 36. The controller 18 may receive signals from the pressure sensors A1-A4 that are indicative of the pressure within the fluid flow circuit 12 and, if a signal indicates a low- or highpressure condition, the controller 18 may initiate an alarm or error condition to alert an operator to the condition and/or to attempt to bring the pressure to an acceptable level without operator intervention.

The blood processing device 10 may also include a plurality of pumps P1-P6 (which are collectively referred to herein as the "pump system" of the blood processing device 10) to cause fluid to flow through the fluid flow circuit 12. The pumps P1-P6 may be differently or similarly configured and/or function similarly or differently from each other. In the illustrated embodiment, the pumps P1-P6 are configured as peristaltic pumps, which may be generally configured as described in U.S. Patent No. 5,868,696. Each pump P1-P6 engages a different tubing loop extending from a side surface of the flow control cassette and may be selectively operated under command of the controller 18 to cause fluid to flow through a portion of the fluid flow circuit 12. In one embodiment, all or a portion of the cassette station 54 may be capable of translational motion in and out of the case 20 to allow for automatic loading of the tubing loops into the associated pump P1-P6. In another exemplary embodiment, rather than employing peristaltic pumps, pneumatic pumps may be employed, with actuators incorporated into the cassette station 54 interacting with suitably configured portions of the fluid flow circuit 12 (e.g., pump stations of a cassette mounted to the cassette station 54) to convey fluid through the fluid flow circuit 12.

The illustrated blood processing device 10 also includes a centrifuge fluid sensor M1 for determining one or more properties of a fluid flowing into or out of a centrifugal separation chamber 36 mounted within the centrifugal separator 16. If the fluid flowing into the centrifuge separation chamber 36 is whole blood (which may include anticoagulated whole blood), the sensor M1 may be configured to determine the hematocrit of the blood flowing into the centrifuge separation chamber 36. If a fluid flowing out of the centrifuge separation chamber 36 is platelet-rich plasma, the sensor M1 may be configured to determine the platelet concentration of the platelet-rich plasma. The sensor M1 may detect the one or more properties of a fluid by optically monitoring the fluid as it flows through tubing of the fluid flow circuit 12 or by any other suitable approach. The controller 18 may receive signals from the sensor M1 that are indicative of the one or more properties of fluid flowing in fluid flow circuit 12 and use the signals to optimize the separation procedure based upon that property or properties. If the property or properties is/are outside of an acceptable range, then the controller 18 may initiate an alarm or error condition to alert an operator to the condition. A suitable device and method for monitoring hematocrit and/or platelet concentration is described in U.S. Patent No. 6,419,822 (which is incorporated herein by reference), but it should be understood that a different approach may also be employed for monitoring hematocrit and/or platelet concentration of fluid flowing through a fluid flow circuit.

The illustrated blood processing device 10 further includes a spinner outlet sensor M2, which accommodates tubing of the fluid flow circuit 12 that flows a separated fluid component out of the spinning membrane separator 26. The spinner outlet sensor M2 monitors the separated fluid component to determine one or more properties thereof, and may do so by optically monitoring the separated fluid component as it flows through the tubing or by any other suitable approach. In one embodiment, separated plasma flows through the tubing, in which case the spinner outlet sensor M2 may be configured to determine the amount of cellular blood components in the plasma and/or whether the plasma is hemolytic and/or lipemic. This may be done using an optical monitor of the type described in U.S. Patent No. 8,556,793 (which is hereby incorporated herein by reference) that measures the optical density of the fluid in the associated tubing, or by any other suitable device and/or method.

The illustrated blood processing device 10 also includes an air detector M3 (e.g., an ultrasonic bubble detector), which accommodates tubing of the fluid flow circuit 12 that flows fluid to a recipient. It may be advantageous to prevent air from reaching the recipient, whether a human recipient (e.g., the same human that serves as the blood source) or a non-human recipient (e.g., a storage bag or container), so the air detector M3 may transmit signals to the controller 18 that are indicative of the presence or absence of air in the tubing. If the signal is indicative of air being present in the tubing, the controller 18 may initiate an alarm or error condition to alert an operator to the condition and/or to take corrective action to prevent the air from reaching the recipient (e.g., by reversing the flow of fluid through the tubing or diverting flow to a vent location).

In one embodiment, the blood processing device 10 may include a platelet concentrate sensor M4 (Figs. 2 and 3). The platelet concentrate sensor M4 may be incorporated into the blood processing device 10 or may be an independent sensor. The platelet concentrate sensor M4 may be configured to determine the concentration of a concentrated platelet product exiting the spinning membrane separator 26. The sensor M4 may detect the concentration of the concentrated platelet product by optically monitoring the fluid as it flows through tubing of the fluid flow circuit 12 or by any other suitable approach.

The generally vertical portion 24 of the case 20 may include a plurality of volume measurement systems W1-W6 (six are shown, but more or fewer may be provided), each configured to be associated with one or more fluid containers F1-F7 of the fluid flow circuit 12 (Fig. 2). Each volume measurement system W1-W6 is configured to work in combination with the controller 18 to measure a current volume of fluid within an associated fluid container F1-F7 and to calculate a change in that volume between two or more points in time. The individual volume measurement systems W1-W6 may be variously configured without departing from the scope of the present disclosure, which may include two or more of the volume measurement systems W1-W6 being differently configured. In one exemplary embodiment, a volume measurement system W1-W6 may be configured as or include a weight scale configured to support and measure the weight of a fluid within an associated fluid container F1-F7 (with the measured weight being converted to a volume by a component of the volume measurement system W1-W6 or by the controller 18). In another exemplary embodiment, a volume measurement system W1-W6 may include one or more sensors configured to detect a volume and/or a change in volume of a fluid within an associated fluid container F1-F7. Volume measurement systems including additional components (e.g., both a weight scale and a sensor) and/or alternative components may also be employed without departing from the scope of the present disclosure.

Regardless of its particular configuration, each volume measurement system W1-W6 transmits to the controller 18 a signal that is indicative of the volume of the fluid within the associated container F1-F7 to track the change of volume during the course of a procedure. This allows the controller 18 to process the incremental volume changes to derive fluid processing volumes and flow rates and subsequently generate signals to control processing events based, at least in part, upon the derived processing volumes. For example, the controller 18 may diagnose leaks and obstructions in the fluid flow circuit 12 and alert an operator.

The illustrated case 20 is also provided with a plurality of hooks or supports H1 and H2 that may support various components of the fluid flow circuit 12 or other suitably sized and configured objects.

### D. Controller

According to an aspect of the present disclosure, the blood processing device 10 includes a controller 18, which is suitably configured and/or programmed to control operation of the blood processing device 10. In one embodiment, the controller 18 comprises a main processing unit (MPU), which can comprise, e.g., a Pentium^{™} type microprocessor made by Intel Corporation, although other types of conventional microprocessors can be used. In one embodiment, the controller 18 may be mounted inside the generally vertical portion 24 of the case 20, adjacent to or incorporated into an operator interface station (e.g., a touchscreen). In other embodiments, the controller 18 and operator interface station may be associated with the generally horizontal portion 22 or may be incorporated into a separate device that is connected (either physically, by a cable or the like, or wirelessly) to the blood processing device 10.

The controller 18 is configured and/or programmed to execute at least one biological fluid processing procedure but, more advantageously, is configured and/or programmed to execute a variety of different biological fluid processing procedures. For example, the controller 18 may be configured and/or programmed to carry out one or more of the following: a double unit red blood cell collection procedure, a plasma collection procedure, a plasma/red blood cell collection procedure, a red blood cell/platelet/plasma collection procedure, a platelet collection procedure, and a platelet/plasma collection procedure.

More particularly, in carrying out these fluid processing procedures, the controller 18 is configured and/or programmed to control one or more of the following tasks: drawing fluid into a fluid flow circuit 12 mounted to the blood processing device 10, conveying fluid through the fluid flow circuit 12 to a location for separation (i.e., into a spinning membrane separator 26 or centrifugal separation chamber 36 of the fluid flow circuit 12), separating the fluid into two or more components as desired, and conveying the separated components into storage containers, to a second location for further separation (e.g., into whichever of the spinning membrane separator 26 and centrifugal separation chamber 36 that was not used in the initial separation stage), or to a recipient (which may be a source from which the fluid was originally drawn).

This may include instructing the spinning membrane separator drive unit 14 and/or the centrifugal separator 16 to operate at a particular rotational speed and instructing a pump P1-P6 to convey fluid through a portion of the fluid flow circuit 12 at a particular flow rate. Hence, while it may be described herein that a particular component of the blood processing device 10 (e.g., the spinning membrane separator drive unit 14 or the centrifugal separator 16) performs a particular function, it should be understood that that component is being controlled by the controller 18 to perform that function.

Before, during, and after a procedure, the controller 18 may receive signals from various components of the blood processing device 10 (e.g., the pressure sensors A1-A4 and sensors M1-M4) to monitor various aspects of the operation of the blood processing device 10 and characteristics of the fluid and separated fluid components as they flow through the fluid flow circuit 12. If the operation of any of the components and/or one or more characteristics of the fluid or separated fluid components is outside of an acceptable range, then the controller 18 may initiate an alarm or error condition to alert the operator and/or take action to attempt to correct the condition. The appropriate corrective action will depend upon the particular error condition and may include action that is carried out with or without the involvement of an operator.

For example, the controller 18 may include an interface control module, which receives signals from the light detector 52 of the interface monitoring system. The signals that the controller 18 receives from the light detector 52 are indicative of the location of an interface between the separated fluid components within the centrifugal separation chamber 36. If the controller 18 determines that the interface is in the wrong location, then it can issue commands to the appropriate components of the blood processing device 10 to modify their operation so as to move the interface to the proper location. For example, the controller 18 may instruct one of the pumps P1-P6 to cause fluid to flow into the centrifugal separation chamber 36 at a different rate and/or for a separated fluid component to be removed from the centrifugal separation chamber 36 at a different rate and/or for the centrifugal separation chamber 36 to be spun at a different speed by the centrifugal separator 16.

If provided, an operator interface station associated with the controller 18 allows the operator to view on a screen or display (in alpha-numeric format and/or as graphical images) information regarding the operation of the system. The operator interface station also allows the operator to select applications to be executed by the controller 18, as well as to change certain functions and performance criteria of the system. If configured as a touchscreen, the screen of the operator interface station can receive input from an operator via touch-activation. Otherwise, if the screen is not a touchscreen, then the operator interface station may receive input from an operator via a separate input device, such as a computer mouse or keyboard. It is also within the scope of the present disclosure for the operator interface station to receive input from both a touchscreen and a separate input device, such as a keypad.

### II. The Disposable Fluid Flow Circuit

As for the fluid flow circuit or flow set 12 (Figs. 2 and 3), it is intended to be a sterile, single use, disposable item. Before beginning a given fluid processing procedure, the operator loads various components of the fluid flow circuit 12 in the case 20 in association with the blood processing device 10. The controller 18 implements the procedure based upon preset protocols, taking into account other input from the operator. Upon completing the procedure, the operator removes the fluid flow circuit 12 from association with the blood processing device 10. The portions of the fluid flow circuit 12 holding the collected fluid component or components (e.g., collection containers or bags) are removed from the case 20 and retained for storage, transfusion, or further processing. The remainder of the fluid flow circuit 12 is removed from the case 20 and discarded.

In the illustrated embodiment, the fluid flow circuit 12 includes a cassette, to which the other components of the fluid flow circuit 12 are connected by flexible tubing. The other components may include a plurality of fluid containers F1-F7. In the context of the present disclosure these containers include an anticoagulant container F1, a saline container F2, an in-process container F3, a return container F4, a plasma collection container F5, a platelet collection container F6, and an (optional) additive container F7. The illustrated flow circuit 12 further includes one or more fluid source access devices (e.g., a connector for accessing blood within a fluid container or a phlebotomy needle), a spinning membrane separator 26 and a centrifugal separation chamber 36.

The flow control cassette provides a centralized, programmable, integrated platform for all the pumping and many of the valving functions required for a given fluid processing procedure. In one embodiment, the cassette is similarly configured to the cassette of U.S. Patent No. 5,868,696, but is adapted to include additional components (e.g., more tubing loops) and functionality.

In use, the cassette is mounted to the cassette station 54 of the blood processing device 10 so as to align each sensor station with an associated pressure sensor A1-A4 of the cassette station 54 and its valve stations with an associated valve V1-V9. Each valve station may define one or more ports that allow fluid communication between the valve station and another interior cavity of the cassette (e.g., a flow path). As described above, each valve V1-V9 is movable under command of the controller 18 to move between a plurality of positions (e.g., between a retracted or lowered position and an actuated or raised position) to selectively contact the valve stations of the cassette. In the actuated position, a valve V1-V9 engages the associated valve station to close one or more of its ports to prevent fluid flow therethrough. In the retracted position, a valve V1-V9 is disengaged from the associated valve station (or less forcefully contacts the associated valve station than when in the actuated position) to open one or more ports associated with the valve station, thereby allowing fluid flow therethrough.

A plurality of tubing loops extend from the side surface of the cassette to interact with pumps P1-P6 of the blood processing device 10. The different pumps P1-P6 may interact with the tubing loops of the cassette to perform different tasks during a procedure, but in the context of the present disclosure, a different one of the pumps P1-P6 may be configured to serve as an anticoagulant pump P1, a source pump P2, a centrifuge pump P3, an outlet pump P4, a recirculation pump P5, and a plasma pump P6. If the pumps P1-P6 are differently configured (e.g., if they are configured as pneumatic pumps), then the cassette may be differently configured (e.g., with pump stations aligned with pneumatic pump actuators) to allow for the pumps P1-P6 to convey fluid through the cassette.

Additional tubing extends from the side surface of the cassette to connect to the other components of the fluid flow circuit 12, such as the various fluid containers F1-F7, the spinning membrane separator 26, and the centrifugal separation chamber 36. The tubing connected to the centrifugal separator chamber 36 (which includes one inlet tube and two outlet tubes) may be aggregated into an umbilicus.

Various additional components may be incorporated into the tubing leading out of the cassette or into one of the cavities of the cassette. For example, a manual clamp 56 may be associated with a line or lines leading to the fluid source, a return line filter 58 (e.g., a microaggregate filter) may be associated with a line leading to a fluid recipient, and/or an air trap 62 may be positioned on a line upstream of the centrifugal separation chamber 36.

### III. Exemplary Blood Processing Procedure

An exemplary blood processing procedure according to the present disclosure will now be described.

Prior to processing, an operator selects the desired protocol (e.g., using an operator interface station, if provided), which informs the controller 18 of the manner in which it is to control the other components of the blood processing device 10 during the procedure. This may include first selecting one of a plurality of possible procedures that the system is capable of executing and then, after selecting the nature of the procedure, selecting one or more parameters to be in effect during the procedure. For example, this may include selecting a platelet collection procedure from among a variety of blood separation procedures and then selecting a total volume of blood to be processed or a target volume of platelets to be collected during the procedure. If the fluid source is a living source (e.g., a donor or patient), the operator may proceed to enter various parameters, such as the sex/height/weight of the source. In one embodiment, the operator may also enter one or more characteristics of the fluid to be processed, such as a platelet pre-count.

Once the controller 18 has received the necessary input, it may proceed to instruct the operator to mount the fluid flow circuit 12 to the blood processing device 10. If there are any fluid containers (e.g., a platelet additive solution container) that are not integrally formed with the fluid flow circuit 12, they may be connected to the fluid flow circuit 12 (e.g., by piercing a septum of a tube of the fluid flow circuit 12 or via a luer connector), with the fluid flow circuit 12 then being mounted to the blood processing device 10 (including the fluid containers F1-F7 being associated with the volume measurement systems W1-W6, as appropriate). In one exemplary embodiment, each volume measurement system W1-W6 includes a weight scale associated with a hook from which a fluid container may be hung. In another exemplary embodiment, at least one of the volume measurement systems W1-W6 includes a weight scale associated with a horizontal platform or surface, with a container being placed onto the platform or surface for support while the weight scale sends signals indicative of the weight of the container (and its contents) to be sent to the controller 18 throughout the course of a procedure. In other embodiments, a fluid container may be associated with a volume measurement system omitting a weight scale, but including other means for measuring the volume of fluid within the container (e.g., one or more sensors).

Once the fluid flow circuit 12 has been fully mounted to the blood processing device 10, the controller 18 may proceed with an integrity check of the fluid flow circuit 12 to ensure that the various components of the fluid flow circuit 12 are properly connected and functioning. Following a successful integrity check, the fluid source is connected to the fluid flow circuit 12 (e.g., by connecting to a container of previously collected fluid or by phlebotomizing a donor), and the fluid flow circuit 12 may be primed (e.g., using saline pumped from a saline container F2 by operation of one or more of the pumps P1-P6 of the blood processing device 10).

After the fluid flow circuit 12 has been primed, fluid processing may begin. In a draw phase of an exemplary platelet collection procedure (which is illustrated in Fig. 3), blood is drawn into the fluid flow circuit 12 from a blood source. If the blood source is a donor, then blood may be drawn into the fluid flow circuit 12 through a single needle that is connected to the cassette by line L1. Line L1 may include a manual clamp 56 that may initially be in a closed position to prevent fluid flow through line L1. When processing is to begin, an operator may move the manual clamp 56 from its closed position to an open position to allow fluid flow through line L1.

The blood is drawn into line L1 by the source pump P2 of the blood processing device 10. Anticoagulant from the anticoagulant container F1 may be drawn through line L2 under action of the anticoagulant pump P1 and added to the blood at a junction of lines L1 and L2.

In the illustrated embodiment, valve V10 is open to allow anticoagulated blood to flow through line L3 and a cassette sensor station associated with pressure sensor A1, while valve V11 is closed to prevent fluid flow through line L4. If the blood source is a living body (e.g., a donor), the pressure sensor A1 may communicate with the controller 18 to monitor the pressure within the vein of the blood source.

The cassette includes two valve stations downstream of the source pump P2, with valve V2 being closed to prevent flow through line L5 and valve V1 being open to allow flow through line L6. A portion of the blood is directed through line L7 and a cassette sensor station associated with pressure sensor A3 to the in-process container F3 and the remainder is directed through line L8 toward the centrifuge pump P3, which controls the amount of blood that is directed to the centrifugal separation chamber 36 instead of the in-process container F3. In particular, the flow rate of the source pump P2 is greater than the flow rate of the centrifuge pump P3, with the difference therebetween being equal to the flow rate of blood into the in-process container F3. The flow rates may be selected such that the in-process container F3 is partially or entirely filled with blood at the end of the draw phase.

The blood pumped through line L8 by the centrifuge pump P3 passes through line 9, an air trap 62, a cassette sensor station associated with pressure sensor A2 (which works in combination with the controller 18 of the blood processing device 10 to monitor the pressure in the centrifugal separation chamber 36), and the centrifuge fluid sensor M1 before reaching the centrifugal separation chamber 36 of the fluid flow circuit 12. The centrifuge fluid sensor M1 may operate in combination with the controller 18 to determine a characteristic of the blood flowing into the centrifugal separation chamber 36, such as the hematocrit of the blood.

The centrifugal separator 16 of the blood processing device 10 manipulates the centrifugal separation chamber 36 to separate the blood in the centrifugal separation chamber 36 into platelet-rich plasma and packed red blood cells. In one embodiment, the centrifugal separation chamber 36 is rotated nominally at 4,500 rpm, but the particular rotational speed may vary depending on the flow rates of fluids into and out of the centrifugal separation chamber 36.

The packed red blood cells exit the centrifugal separation chamber 36 via line L10 and flow through line L11 into the return container F4. The packed red blood cells travel through the centrifuge fluid sensor M1 after they exit the centrifugal separation chamber 36, with the centrifuge fluid sensor M1 operating in combination with the controller 8 to determine a characteristic of the red blood cells. In one embodiment, the centrifuge fluid sensor M1 operates in combination with the controller 18 to determine the hematocrit of the packed red blood cells exiting the centrifugal separation chamber 36.

Platelet-rich plasma is drawn out of the centrifugal separation chamber 36 via line L12 by the combined operation of the recirculation and outlet pumps P5 and P4 of the blood processing device 10. The platelet-rich plasma travels through the centrifuge fluid sensor M1 and line L12 until it reaches a junction, which splits into lines L13 and L14. The centrifuge fluid sensor M1 may operate in combination with the controller 18 to determine a characteristic of the platelet-rich plasma, such as the concentration of platelets in the platelet-rich plasma.

The recirculation pump P5 is associated with line L13 and redirects a portion of the platelet-rich plasma to a junction, where it mixes with blood in line L8 that is being conveyed into the centrifugal separation chamber 36 by the centrifuge pump P3. Recirculating a portion of the platelet-rich plasma into the centrifugal separation chamber 36 with inflowing blood decreases the hematocrit of the blood entering the centrifugal separation chamber 36, which may improve separation efficiency. By such an arrangement, the flow rate of the fluid entering the centrifugal separation chamber 36 is equal to the sum of the flow rates of the centrifuge pump P3 and the recirculation pump P5. As the platelet-rich plasma drawn out of the centrifugal separation chamber 36 into line L13 by the recirculation pump P5 is immediately added back into the centrifugal separation chamber 36, the bulk or net platelet-rich plasma flow rate out of the centrifugal separation chamber 36 is equal to the flow rate of the outlet pump P4.

Line L14 ends at a junction, where it joins with lines L15 and L16. Valve V6 is closed to prevent fluid flow through line L16, thereby directing the separated platelet-rich plasma to the spinning membrane separator 26 via line L15. Before reaching the spinning membrane separator 26, the portion of the platelet-rich plasma conveyed through line L15 passes through a cassette sensor station associated with pressure sensor A4, which may monitor the pressure of the spinning membrane separator 26.

While valve V6 is typically closed, it may be selectively opened to divert all or a portion of the platelet-rich plasma from line L14 into and through line L16 and to the return container F4, if necessary. An example would be at the start of a procedure when separation is initializing and platelets are not yet exiting the centrifugal separation chamber 36, in which case the fluid conveyed through line L14 by the outlet pump P4 could be diverted to the return container F4.

The spinning membrane separator drive unit 14 of the blood processing device 10 manipulates the spinning membrane separator 26 to separate the platelet-rich plasma into platelet-poor plasma ("plasma") and platelet concentrate ("platelets"). Plasma is pumped out of the spinning membrane separator 26 via line L17 by the plasma pump P6 of the blood processing device 10. Valves V5, V6, V8, and V9 are closed to direct the separated plasma along line L18, through valve V4, and into the return container F4 (with the separated red blood cells). On the way to the return container F4, the plasma passes through spinner outlet sensor M2, which may cooperate with the controller 18 to determine one or more characteristics of the plasma, such as the amount of cellular blood components in the plasma and/or whether the plasma is hemolytic and/or lipemic.

The platelet concentrate is conveyed out of the spinning membrane separator 26 via line L19. The platelet concentrate passes through the platelet concentrate sensor M4, which may work in combination with the controller 18 to determine one or more characteristics of the platelet concentrate, such as the concentration of platelets in the platelet concentrate.

There is no pump associated with line L19, so instead the flow rate at which the platelets exit the spinning membrane separator 26 is equal to the difference between the flow rates of the outlet pump P4 and plasma pump P6. Valve V8 is closed to prevent fluid flow through the line L20, thereby directing the flow of platelets along line L19, through the platelet concentrate sensor M4 and valve V7, and into the platelet collection container F6. Valve V8 may be selectively opened to allow fluid flow through line L20 and to a junction, where it joins the plasma flowing through line L18 to the return container F4, if necessary.

Depending on the volume of platelets to be collected, the above-described draw stage may be repeated, with draw stages being alternated with return stages in which blood from the in-process container F3 is separated in the centrifugal separation chamber 36 while previously collected blood components in the return container F4 are returned to the blood source. During such return stages, the separated red blood cells and platelet-rich plasma may be variously routed through the fluid flow circuit 12, typically with an additional volume of platelets being collected in the platelet collection container F6 after being separated from platelet-poor plasma in the spinning membrane separator 26 (as during the draw stage). A platelet additive solution from the additive container F7 may be added to the collected platelets in the platelet collection container F6 before ending the procedure.

### IV. Automatic Adjustment Of Light Intensity Of Optical Detection Assembly

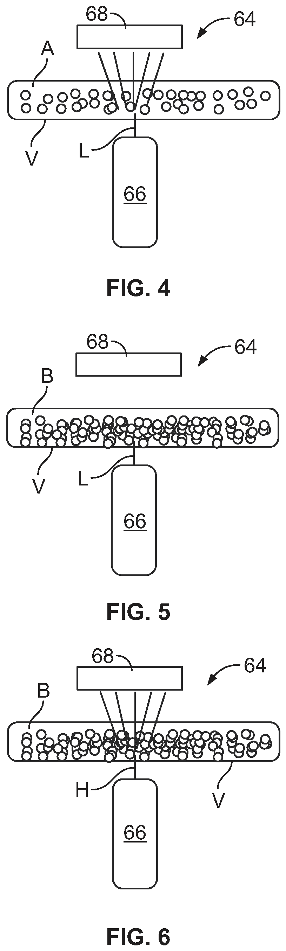

As described above, the interface monitoring system of the blood processing device 10 includes an optical detection assembly having a light source and a light detector. At least one of the sensors M1, M2, M3, and M4 of the blood processing device 10 may also include or be configured as an optical detection assembly having a light source and a light detector. Figs. 4-6 illustrate an exemplary optical detection assembly 64 (which is suitable for incorporation into the interface monitoring system or any one of the sensors M1, M2, M3, and M4), with the optical detection assembly 64 having a light source 66 and a light detector 68. The light source 66 may be variously configured without departing from the scope of the present disclosure (which may include the light source 66 being configured as a light bulb or a light-emitting diode or a laser, for example) and is configured to emit light toward a fluid in a vessel "V" or region of a fluid flow circuit 12. At least a portion of the light from the light source 66 may be received by the light detector 68, which may be variously configured without departing from the scope of the present disclosure (which may include the light detector 68 being configured as a photodiode or as an array of detectors, such as a linear array, for example). When the light detector 68 has received at least a portion of the light emitted by the light source 66, it transmits a signal to the controller 18 of the blood processing device 10 that is indicative of the intensity of the light received by the light detector 68. For example, the signal transmitted from the light detector 68 to the controller 18 may have a relatively low voltage when the light detector 68 has received light having a relatively low intensity, whereas the signal transmitted from the light detector 68 to the controller 18 may have a relatively high voltage when the light detector 68 has received light having a relatively high intensity.

When the light emitted by the light source 66 interacts with a fluid being monitored by the optical detection assembly 64, the intensity of the portion of that light that is ultimately received by the light detector 68 will be affected by the cellular composition of the fluid. Typically, fluids having a relatively high cellular concentration (e.g., platelet-rich plasma having a relatively high concentration of platelets or packed red cells having a relatively high hematocrit or concentration of red blood cells) will result in the light detector 68 receiving light having a lower intensity than the light received by the light detector 68 when a fluid having a relatively low cellular concentration (e.g., platelet-poor plasma having a relatively low concentration of platelets or whole blood having a relatively low hematocrit or concentration of red blood cells) is being monitored by the optical detection assembly 64. Thus, by comparing the intensity of the light emitted by the light source 66 to the intensity of the light received by the light detector 68 (which may be determined based on the signal transmitted by the light detector 68), the controller 18 may determine the cellular concentration of the fluid being monitored by the optical detection assembly 64 (e.g., by referring to a database correlating the signal from the light detector 68 to the cellular concentration of the fluid being monitored). One exemplary approach to determining the cellular concentration of a fluid using an optical detection assembly is described in U.S. Patent Application Publication No. 2023/0243746, which is hereby incorporated herein by reference. While optical monitoring may most typically be used to determine the platelet concentration or the red blood cell concentration of a fluid, it should be understood that the techniques described herein are not limited to detection of the concentration of any particular substances in a fluid.

If the cellular concentration of a fluid being monitored by the optical detection assembly 64 is sufficiently high, it is possible that the light detector 68 will not receive any of the light from the light source 66 or at least not enough light to allow for the controller 18 to determine the cellular concentration of the fluid. For example, Fig. 4 shows the optical detection assembly 64 monitoring a fluid "A" having a relatively low cellular concentration, with a portion of the light "L" emitted by the light source 66 ultimately reaching the light detector 68. The output signal from the light detector 68 will be sufficiently strong to allow for the controller 18 to determine the cellular concentration of the fluid A in Fig. 4. On the other hand, Fig. 5 shows the optical detection assembly 64 monitoring a fluid "B" having a relatively high cellular concentration, with none of the light L emitted by the light source 66 reaching the light detector 68. In this situation, the light detector 68 will not be able to transmit a signal that can be used by the controller 18 to determine the cellular concentration of the fluid B. It would, thus, be advantageous for light having a greater intensity to be emitted by the light source 66 when monitoring the high-concentration fluid B so as to ensure that light having sufficient intensity will reach the light detector 68 after interacting with the fluid B.

While light having a relatively high intensity may be advantageous when monitoring fluid having a relatively high cellular concentration, it may be inappropriate when monitoring fluid having a relatively low cellular concentration. More particularly, a light detector may be configured to transmit signals having a maximum level or voltage when it has received light having a certain intensity (which may be referred to as "light detector saturation"). When the light detector receives light having a greater intensity than that, it will still only be capable of transmitting an output signal having the same maximum level or voltage, resulting in an associated controller being incapable of detecting any difference in the intensity of light above a certain level received by the light detector. As described above, fluid having a relatively low cellular concentration will not result in a significant decrease in the intensity of the light from a light source that is ultimately received by a light detector. As such, when the light source emits light having a relatively high intensity toward a fluid having a relatively low cellular concentration, it is possible for the light detector to become saturated (because interaction with the fluid will not reduce sufficiently decrease the intensity of the light) and the controller will not be able to analyze the signal from the light detector to determine the cellular concentration of the fluid. It is, therefore, advantageous for light having a relatively low intensity to be emitted by the light source 66 when monitoring the low-concentration fluid A of Fig. 4 so as to ensure that the light detector 68 does not become saturated.

In view of the foregoing, and in accordance with an aspect of the present disclosure, at least one of the sensors M1, M2, M3, and M4 and/or the interface monitoring system of the blood processing device 10 includes or is configured as an optical detection assembly 64 having a light source 66 that is capable of emitting light at different intensity levels. This may include the light source 66 being configured to emit light at any level between a maximum intensity and a minimum intensity or the light source 66 being configured to emit light at a certain number of different intensity levels (e.g., a "high" intensity level of light and a "low" intensity level of light).

In the embodiment of Figs. 4-6, the light source 66 is emitting a relatively low intensity light L in Figs. 4 and 5, while Fig. 6 shows the light source 66 emitting a relatively high intensity light "H." Fig. 4 illustrates a first fluid A having a relatively low cellular concentration (e.g., platelet-poor plasma having a relatively low concentration of platelets or whole blood having a relatively low concentration of red blood cells), while Figs. 5 and 6 illustrate a second fluid B having a relatively high cellular concentration (e.g., platelet-rich plasma having a relatively high concentration of platelets or packed red blood cells having a relatively high concentration of red blood cells). As shown in Fig. 5, the low-intensity light L is insufficient to provide the light detector 68 with enough light to produce an output signal that can be analyzed by the controller 18, so the controller 18 will control the light source 66 to emit higher intensity light H when the optical detection assembly 64 is monitoring the high-concentration fluid B (Fig. 6) so as to allow the light detector 68 to transmit an output signal that can be analyzed by the controller 18.

This variable light intensity may be implemented by any suitable approach without departing from the scope of the present disclosure. For example, in one exemplary embodiment, a power source associated with the light source 66 may be controlled by the controller 18 to provide the light source 66 with a relatively low level of power in order to cause the light source 66 to emit light L having a relatively low intensity, while being controlled by the controller 18 to provide the light source 66 with a greater level of power in order to cause the light source 66 to emit light H having a relatively high intensity. In another embodiment, the light source 66 may be provided with a filter or screen that is applied in order to selectively cause light emitted by the light source 66 to have a lower intensity than it would in the absence of the filter or screen. In yet another embodiment, the light source 66 may be comprised of a plurality of individual sources (e.g., a plurality of bulbs or light-emitting diodes or lasers), with one or more of the individual sources being inactive when the light source 66 is to emit light at a relatively low intensity.

The controller 18 may be programmed to determine the intensity of light to be emitted by the light source 66 and, upon determining the appropriate intensity, control the light source 66 to emit light at that intensity. In one exemplary embodiment, the controller 18 may determine the appropriate light intensity based on a range in which the cellular concentration of a fluid to be monitored by the optical detection assembly 64 during a blood processing procedure is expected to fall. For example, it may be expected that the platelet concentration of a fluid to be monitored during "steady state" blood separation for platelets stored in plasma will be in the range of 1,000 - 2,000 e3/µL, whereas it may be expected that the platelet concentration of a fluid to be monitored during "steady state" blood separation for platelets stored in a platelet additive solution will be in the range of 3,500 - 4,500 e3/µL. Each expected cellular concentration range may have an associated light intensity associated with it (typically, with a relatively low light intensity for a lower concentration range and a relatively high light intensity for a higher concentration range), with the appropriate light intensity being empirically derived. The expected cellular concentration ranges and corresponding light intensities may be stored in a database, which may be an internal database programmed into the controller 18 or an external database that may be accessed by the controller 18.

Different fluids (or at least fluids having significantly different cellular concentrations) may be present in the same region of a fluid flow circuit 12 during different types of blood processing procedures. For example, platelet-poor plasma may be present in a vessel V of a fluid flow circuit 12 being monitored by an optical detection assembly 64 during a first type of blood processing procedure, whereas platelet-rich plasma may be present in that same vessel V or region during a second type of blood processing procedure. Thus, according to one aspect of the present disclosure, the controller 18 may be programmed to determine an expected range of cellular concentrations for a fluid to be monitored upon being instructed by an operator (or upon otherwise determining) the type of blood processing procedure that is to be executed by the controller 19.

Similarly, different fluids (or at least fluids having significantly different cellular concentrations) may be present in the same region of a fluid flow circuit 12 during different phases of a single blood processing procedure having a plurality of phases. For example, saline or some other (non-blood) priming fluid may be present in a vessel V being monitored by an optical detection assembly 64 during a priming phase of a procedure, while blood or a separated blood component may be in that same vessel V or region during a later phase of the same procedure. In another example, it may be expected that a plasma layer separated from whole blood will have a relatively low platelet concentration during initial processing and a greater platelet concentration once "steady state" separation has been achieved (with the plasma layer being monitored in the same region of a fluid flow circuit 12 by a single optical detection assembly 64). Thus, according to one aspect of the present disclosure, the controller 18 may be programmed to determine an expected range of cellular concentrations for a fluid to be monitored upon determining the current phase of a blood processing procedure being executed by the controller 18.

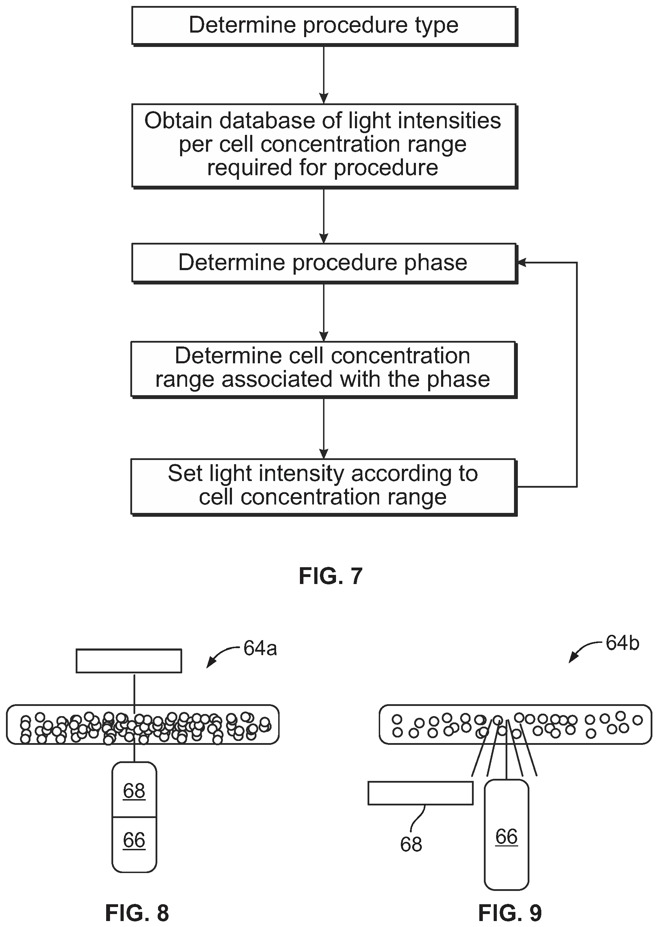

The procedure-based determination of an expected cellular concentration range and the phase-based determination of an expected cellular concentration range may be practiced separately or in combination, with Fig. 7 illustrating an exemplary implementation of the two approaches in combination. According to the exemplary approach shown in Fig. 7, the controller 18 may first determine the type of blood processing procedure that it will be executing (which may include the controller 18 being instructed by an operator to execute a particular type of procedure). Upon determining the procedure type, the controller 18 proceeds to access a database correlating expected ranges of cellular concentrations for fluids to be monitored by one or more optical detection assemblies 64 of the blood processing device 10 during different phases of the procedure to the appropriate intensity of light to be emitted by the light source 66 of each optical detection assembly 64 during each phase. Throughout the course of the procedure, the controller 18 will periodically determine the current phase of the procedure, access the database to determine the expected cellular concentration range for fluid to be monitored during that phase by each optical detection assembly 64, and control the light source 66 of each optical detection assembly 64 to emit light at the appropriate intensity during that phase of the procedure. By such an approach, the controller 18 will operate to automatically change the light intensity emitted by the light source 66 of an optical detection assembly 64 to an appropriate level for different phases of a procedure (or will act to maintain the light intensity at a uniform level for an optical detection assembly 64, if no significant change in the cellular concentration of a fluid being monitored by that optical detection assembly 64 is expected during a blood processing procedure).

While Figs. 4-6 illustrate an optical detection assembly 64 in which a light detector 66 is positioned and configured to receive light emitted through a fluid, it should be understood that the principles described herein are not limited to an optical detection assembly having any particular configuration or mode of operation. For example, Fig. 8 illustrates an optical detection assembly 64a in which a light detector 68 is positioned and configured to receive reflected light from an associated light source 66, while Fig. 9 illustrates an optical detection assembly 64b in which a light detector 68 is positioned and configured to receive light scattered by a fluid being monitored.

### V. Aspects

Aspect 1. A blood processing device comprising: a pump system; a valve system; a controller programmed to control the operation of the pump system and the valve system to execute a blood processing procedure; and an optical detection assembly comprising a light source and a light detector and configured to be controlled by the controller to monitor a fluid during the blood processing procedure, wherein the controller is programmed to determine an expected range of cellular concentrations for the fluid, determine or be provided with an intensity of light to be emitted by the light source during the blood processing procedure based at least in part on the expected range of cellular concentrations for the fluid, control the light source during the blood processing procedure to emit light at said intensity toward the fluid, control the light detector during the blood processing procedure to transmit a signal to the controller based on light from the light source received by the light detector, and determine a cellular concentration of the fluid based at least in part on the signal from the light detector.

Aspect 2. The blood processing device of Aspect 1, wherein the light detector is positioned and configured to receive light from the light source emitted through the fluid.

Aspect 3. The blood processing device of Aspect 1, wherein the light detector is positioned and configured to receive reflected light from the light source.

Aspect 4. The blood processing device of Aspect 1, wherein the light detector is positioned and configured to receive light scattered by the fluid.

Aspect 5. The blood processing device of any one of the preceding Aspects, wherein the controller is programmed to retrieve the intensity of light to be emitted by the light source during the blood processing procedure from an external database including the intensity of light to be emitted by the light source for each of a plurality of expected ranges of cellular concentrations for the fluid.

Aspect 6. The blood processing device of any one of Aspects 1-4, wherein the controller is programmed with an internal database including the intensity of light to be emitted by the light source for each of a plurality of expected ranges of cellular concentrations for the fluid, and the controller is programmed to retrieve the intensity of light to be emitted by the light source during the blood processing procedure from the internal database after determining the expected range of cellular concentrations for the fluid.

Aspect 7. The blood processing device of any one of the preceding Aspects, wherein the controller is programmed to control the operation of the pump system and the valve system to execute a plurality of different blood processing procedures, and the controller is programmed to determine the expected range of cellular concentrations for the fluid based at least in part on which one of the plurality of different blood processing procedures is to be executed.

Aspect 8. The blood processing device of any one of the preceding Aspects, wherein the blood processing procedure includes a plurality of phases, with the expected range of cellular concentrations for the fluid during a first phase of the blood processing procedure being different from the expected range of cellular concentrations for the fluid during a second phase of the blood processing procedure, and the controller is programmed to determine the expected range of cellular concentrations for the fluid during the first phase of the blood processing procedure, determine or be provided with a first intensity of light to be emitted by the light source during the first phase of the blood processing procedure based at least in part on the expected range of cellular concentrations for the fluid during the first phase of the blood processing procedure, control the light source during the first phase of the blood processing procedure to emit light at said first intensity, control the light detector during the first phase of the blood processing procedure to transmit a first signal to the controller based on light from the light source received by the light detector, determine a first cellular concentration of the fluid based at least in part on the first signal from the light detector, determine the expected range of cellular concentrations for the fluid during the second phase of the blood processing procedure, determine or be provided with a second intensity of light to be emitted by the light source during the second phase of the blood processing procedure based at least in part on the expected range of cellular concentrations for the fluid during the second phase of the blood processing procedure, control the light source during the second phase of the blood processing procedure to emit light at said second intensity, control the light detector during the second phase of the blood processing procedure to transmit a second signal to the controller based on light from the light source received by the light detector, and determine a second cellular concentration of the fluid based at least in part on the second signal from the light detector.

Aspect 9. The blood processing device of any one of the preceding Aspects, wherein the cellular concentration for the fluid is a platelet concentration.

Aspect 10. The blood processing device of any one of Aspects 1-8, wherein the cellular concentration for the fluid is a red blood cell concentration.

Aspect 11. A controller-implemented method of executing a blood processing procedure using a blood processing device comprising a pump system, a valve system, and an optical detection assembly comprising a light source and a light detector and configured to be controlled by the controller to monitor a fluid during the blood processing procedure, the method comprising the controller: determining an expected range of cellular concentrations for the fluid; determining or being provided with an intensity of light to be emitted by the light source during the blood processing procedure based at least in part on the expected range of cellular concentrations for the fluid; controlling the light source during the blood processing procedure to emit light at said intensity toward the fluid; controlling the light detector during the blood processing procedure to transmit a signal to the controller based on light from the light source received by the light detector; and determining a cellular concentration of the fluid based at least in part on the signal from the light detector.

Aspect 12. The method of Aspect 11, further comprising the light detector receiving light from the light source emitted through the fluid.

Aspect 13. The method of Aspect 11, further comprising the light detector receiving reflected light from the light source.

Aspect 14. The method of Aspect 11, further comprising the light detector receiving light scattered by the fluid.

Aspect 15. The method of any one of Aspects 11-14, wherein said determining or being provided with an intensity of light to be emitted by the light source during the blood processing procedure based at least in part on the expected range of cellular concentrations for the fluid includes the controller retrieving the intensity of light to be emitted by the light source during the blood processing procedure from an external database including the intensity of light to be emitted by the light source for each of a plurality of expected ranges of cellular concentrations for the fluid.

Aspect 16. The method of any one of Aspects 11-14, wherein the controller is programmed with an internal database including the intensity of light to be emitted by the light source for each of a plurality of expected ranges of cellular concentrations for the fluid, and said determining or being provided with an intensity of light to be emitted by the light source during the blood processing procedure based at least in part on the expected range of cellular concentrations for the fluid includes the controller retrieving the intensity of light to be emitted by the light source during the blood processing procedure from the internal database after determining the expected range of cellular concentrations for the fluid.

Aspect 17. The method of any one of Aspects 11-16, wherein the controller is programmed to control the operation of the pump system and the valve system to execute a plurality of different blood processing procedures, and said determining an expected range of cellular concentrations for the fluid includes the controller determining the expected range of cellular concentrations for the fluid based at least in part on which one of the plurality of different blood processing procedures is to be executed.

Aspect 18. The method of any one of Aspects 11-17, wherein the blood processing procedure includes a plurality of phases, with the expected range of cellular concentrations for the fluid during a first phase of the blood processing procedure being different from the expected range of cellular concentrations for the fluid during a second phase of the blood processing procedure, and the method includes the controller determining the expected range of cellular concentrations for the fluid during the first phase of the blood processing procedure, determining or being provided with a first intensity of light to be emitted by the light source during the first phase of the blood processing procedure based at least in part on the expected range of cellular concentrations for the fluid during the first phase of the blood processing procedure, controlling the light source during the first phase of the blood processing procedure to emit light at said first intensity, controlling the light detector during the first phase of the blood processing procedure to transmit a first signal to the controller based on light from the light source received by the light detector, determining a first cellular concentration of the fluid based at least in part on the first signal from the light detector, determining the expected range of cellular concentrations for the fluid during the second phase of the blood processing procedure, determining or being provided with a second intensity of light to be emitted by the light source during the second phase of the blood processing procedure based at least in part on the expected range of cellular concentrations for the fluid during the second phase of the blood processing procedure, controlling the light source during the second phase of the blood processing procedure to emit light at said second intensity, controlling the light detector during the second phase of the blood processing procedure to transmit a second signal to the controller based on light from the light source received by the light detector, and determining a second cellular concentration of the fluid based at least in part on the second signal from the light detector.

Aspect 19. The method of any one of Aspects 11-18, wherein the cellular concentration for the fluid is a platelet concentration.

Aspect 20. The method of any one of Aspects 11-18, wherein the cellular concentration for the fluid is a red blood cell concentration.

It will be understood that the embodiments described above are illustrative of some of the applications of the principles of the present subject matter. Numerous modifications may be made by those skilled in the art without departing from the spirit and scope of the claimed subject matter, including those combinations of features that are individually disclosed or claimed herein. For these reasons, the scope hereof is not limited to the above description but is as set forth in the following claims, and it is understood that claims may be directed to the features hereof, including as combinations of features that are individually disclosed or claimed herein.

## Claims

1. A blood processing device (10) comprising:
a pump system (P1-P6);
a valve system (V1-V11);
a controller (18) programmed to control the operation of the pump system (P1-P6) and the valve system (V1-V11) to execute a blood processing procedure; and
an optical detection assembly (64, 64a, 64b) comprising a light source (66) and a light detector (68) and configured to be controlled by the controller (18) to monitor a fluid during the blood processing procedure, wherein the controller (18) is programmed to
determine an expected range of cellular concentrations for the fluid,
determine or be provided with an intensity of light to be emitted by the light source (66) during the blood processing procedure based at least in part on the expected range of cellular concentrations for the fluid,
control the light source (66) during the blood processing procedure to emit light at said intensity toward the fluid,
control the light detector (68) during the blood processing procedure to transmit a signal to the controller (18) based on light from the light source (66) received by the light detector (68), and
determine a cellular concentration of the fluid based at least in part on the signal from the light detector (68).

2. The blood processing device (10) of claim 1, wherein the light detector (68) is positioned and configured to receive light from the light source (66) emitted through the fluid.

3. The blood processing device (10) of claim 1, wherein the light detector (68) is positioned and configured to receive reflected light from the light source (66).

4. The blood processing device (10) of claim 1, wherein the light detector (68) is positioned and configured to receive light scattered by the fluid.

5. The blood processing device (10) of any one of the preceding claims, wherein the controller (18) is programmed to retrieve the intensity of light to be emitted by the light source (66) during the blood processing procedure from an external database including the intensity of light to be emitted by the light source (66) for each of a plurality of expected ranges of cellular concentrations for the fluid.

6. The blood processing device (10) of any one of claims 1-4, wherein
the controller (18) is programmed with an internal database including the intensity of light to be emitted by the light source (66) for each of a plurality of expected ranges of cellular concentrations for the fluid, and
the controller (18) is programmed to retrieve the intensity of light to be emitted by the light source (66) during the blood processing procedure from the internal database after determining the expected range of cellular concentrations for the fluid.

7. The blood processing device (10) of any one of the preceding claims, wherein
the controller (18) is programmed to control the operation of the pump system (P1-P6) and the valve system (V1-V11) to execute a plurality of different blood processing procedures, and
the controller (18) is programmed to determine the expected range of cellular concentrations for the fluid based at least in part on which one of the plurality of different blood processing procedures is to be executed.

8. The blood processing device (10) of any one of the preceding claims, wherein
the blood processing procedure includes a plurality of phases, with the expected range of cellular concentrations for the fluid during a first phase of the blood processing procedure being different from the expected range of cellular concentrations for the fluid during a second phase of the blood processing procedure, and
the controller (18) is programmed to
determine the expected range of cellular concentrations for the fluid during the first phase of the blood processing procedure,
determine or be provided with a first intensity of light to be emitted by the light source (66) during the first phase of the blood processing procedure based at least in part on the expected range of cellular concentrations for the fluid during the first phase of the blood processing procedure,
control the light source (66) during the first phase of the blood processing procedure to emit light at said first intensity,
control the light detector (68) during the first phase of the blood processing procedure to transmit a first signal to the controller (18) based on light from the light source (66) received by the light detector (68),
determine a first cellular concentration of the fluid based at least in part on the first signal from the light detector (68),
determine the expected range of cellular concentrations for the fluid during the second phase of the blood processing procedure,
determine or be provided with a second intensity of light to be emitted by the light source (66) during the second phase of the blood processing procedure based at least in part on the expected range of cellular concentrations for the fluid during the second phase of the blood processing procedure,
control the light source (66) during the second phase of the blood processing procedure to emit light at said second intensity,
control the light detector (68) during the second phase of the blood processing procedure to transmit a second signal to the controller (18) based on light from the light source (66) received by the light detector (68), and
determine a second cellular concentration of the fluid based at least in part on the second signal from the light detector (68).

9. The blood processing device (10) of any one of the preceding claims, wherein the cellular concentration for the fluid is a platelet concentration.

10. The blood processing device (10) of any one of claims 1-8, wherein the cellular concentration for the fluid is a red blood cell concentration.

11. A controller-implemented method of executing a blood processing procedure using a blood processing device (10) comprising a pump system (P1-P6), a valve system (V1-V11), and an optical detection assembly (64, 64a, 64b) comprising a light source (66) and a light detector (68) and configured to be controlled by the controller (18) to monitor a fluid during the blood processing procedure, the method comprising the controller (18):
determining an expected range of cellular concentrations for the fluid;
determining or being provided with an intensity of light to be emitted by the light source (66) during the blood processing procedure based at least in part on the expected range of cellular concentrations for the fluid;
controlling the light source (66) during the blood processing procedure to emit light at said intensity toward the fluid;
controlling the light detector (68) during the blood processing procedure to transmit a signal to the controller (18) based on light from the light source (66) received by the light detector (68); and
determining a cellular concentration of the fluid based at least in part on the signal from the light detector (68).

12. The method of claim 11, wherein said determining or being provided with an intensity of light to be emitted by the light source (66) during the blood processing procedure based at least in part on the expected range of cellular concentrations for the fluid includes the controller (18) retrieving the intensity of light to be emitted by the light source (66) during the blood processing procedure from an external database including the intensity of light to be emitted by the light source (66) for each of a plurality of expected ranges of cellular concentrations for the fluid.

13. The method of claim 11, wherein
the controller (18) is programmed with an internal database including the intensity of light to be emitted by the light source (66) for each of a plurality of expected ranges of cellular concentrations for the fluid, and
said determining or being provided with an intensity of light to be emitted by the light source (66) during the blood processing procedure based at least in part on the expected range of cellular concentrations for the fluid includes the controller (18) retrieving the intensity of light to be emitted by the light source (66) during the blood processing procedure from the internal database after determining the expected range of cellular concentrations for the fluid.

14. The method of any one of claims 11-13, wherein
the controller (18) is programmed to control the operation of the pump system (P1-P6) and the valve system (V1-V11) to execute a plurality of different blood processing procedures, and
said determining an expected range of cellular concentrations for the fluid includes the controller (18) determining the expected range of cellular concentrations for the fluid based at least in part on which one of the plurality of different blood processing procedures is to be executed.

15. The method of any one of claims 11-14, wherein
the blood processing procedure includes a plurality of phases, with the expected range of cellular concentrations for the fluid during a first phase of the blood processing procedure being different from the expected range of cellular concentrations for the fluid during a second phase of the blood processing procedure, and
the method includes the controller (18)
determining the expected range of cellular concentrations for the fluid during the first phase of the blood processing procedure,
determining or being provided with a first intensity of light to be emitted by the light source (66) during the first phase of the blood processing procedure based at least in part on the expected range of cellular concentrations for the fluid during the first phase of the blood processing procedure,
controlling the light source (66) during the first phase of the blood processing procedure to emit light at said first intensity,
controlling the light detector (68) during the first phase of the blood processing procedure to transmit a first signal to the controller (18) based on light from the light source (66) received by the light detector (68),
determining a first cellular concentration of the fluid based at least in part on the first signal from the light detector (68),
determining the expected range of cellular concentrations for the fluid during the second phase of the blood processing procedure,
determining or being provided with a second intensity of light to be emitted by the light source (66) during the second phase of the blood processing procedure based at least in part on the expected range of cellular concentrations for the fluid during the second phase of the blood processing procedure,
controlling the light source (66) during the second phase of the blood processing procedure to emit light at said second intensity,
controlling the light detector (68) during the second phase of the blood processing procedure to transmit a second signal to the controller (18) based on light from the light source (66) received by the light detector (68), and
determining a second cellular concentration of the fluid based at least in part on the second signal from the light detector (68).
